# EUROPEAN PATENT APPLICATION

(11) **EP 4 383 273 A1**
(43) Date of publication of application: **12.06.2024**
(21) Application number: 22306804.0
(22) Date of filing: 07.12.2022
(51) Int. Cl.: G16H 50/20, G16H 50/30

(54) **SYSTEM AND METHODS FOR IDIOPATHIC ARRHYTHMIAS MONITORING AND PREDICTION**

(71) Applicant: BULL SAS, 78340 Les Clayes-Sous-Bois (FR)
(72) Inventor: JIMENEZ, Natalia, Cambridge, CB1 9YH (GB)
(74) Representative: Cabinet Camus Lebkiri

(57) **Abstract**

According to an aspect of the invention, it is provided a method for detecting idiopathic arrythmias of a user, comprising:
• Receiving a first signal (13) acquired by an electrocardiogram sensor (11);
• Determining an occurrence of idiopathic arrythmia in the first signal (13) based on a comparison between said first signal (13) and at least one reference signals; and
• When the occurrence of the idiopathic arrythmia is determined:
∘ Requesting the user to provide information about his/her health state; and
∘ Sending a message based on the first signal (13) and the information about the health state of the user

## Description

### TECHNICAL FIELD

The technical field of the invention is the field of information technology applied to medicine and in particular that of idiopathic arrythmias monitoring.

The present invention concerns a system and methods for assist in the diagnosis and prediction of idiopathic arrythmias as well as monitoring patients already diagnosed.

### BACKGROUND OF THE INVENTION

Patients suffering from idiopathic arrythmias are usually referred around multiple specialty health services seeking for a diagnosis based on their symptoms. It is understood by "idiopathic arrythmia" an abnormality of the heart's rhythm of unknown cause. In most of the cases, diagnosis cannot be made, leading to an unnecessary health care expenditure and, more importantly, leaving these patients undiagnosed, increasing the risk of an anomalous episode.

Idiopathic arrythmias are actually hard to monitor with common procedures such as electrocardiogram (ECG) by machines in physician's offices, or ambulatory ECG such as Holter monitor that performs and records ECG over a 24 hours or 48 hours period. However, due to their idiopathic nature, this kind of arrythmia is very unlikely to be detected in the short monitoring window provided by ECG and ambulatory ECG procedures. Moreover, said device is handed back to the healthcare provider after the monitoring period, and the acquired data is uploaded to a post-processing system to analyse the data. The analysis of said data is therefore not done in real time, which further delays the establishment of a diagnosis, if possible.

A Holter monitor consists in electrodes connected to a portable machine worn at the patient's waist, which could cause a nuisance to patients for sleeping and day-to-day activities. A subcutaneous Holter monitor is 87 % smaller and allows monitoring periods of over 2 years. However, it is invasive, single use and expensive.

Smart wearable devices such as Apple^{™} Watch^{™}, AliveCor^{™} KardiaBand^{™} and KardiaMobile^{™} and ZIO^{™} Patch can alternatively be used for clinical applications but are not adapted for idiopathic arrythmia monitoring *(*Smart wearable devices in cardiovascular care: where we are and how to move forward, K. Bayoumy et al., Nature Reviews, Cardiology, vol. 18, pp. 581-599, August 2021). Despite all these advances in the creation of sophisticated wearable devices and algorithms, fully integrated solutions that would allow the long-term monitoring and real time diagnosis of idiopathic arrythmias do not exist.

Consequently, there is a need for a better detection of idiopathic arrythmias.

### SUMMARY OF THE INVENTION

An object of the invention is a system for aiding the diagnosis, monitoring, and eventually prediction of idiopathic arrythmias. The monitoring is based on machine learning approaches with the option to alert emergency services to provide assistance if needed.

To this end, according to a first aspect of the invention, it is provided a method for detecting idiopathic arrythmias of a user, the method comprising:
- Receiving a first signal acquired by an electrocardiogram sensor;
- Determining an occurrence of idiopathic arrythmia in the first signal based on a comparison between said first signal and at least one reference signals; and
- When the occurrence of the idiopathic arrythmia is determined:
   - Requesting the user to provide information about his/her health status; and
   - Sending a message based on the first signal and the information about the health state of the user.

Thanks to the invention, it is possible to detect an idiopathic arrythmia and to gather information on the health state of the user when the idiopathic arrythmia is detected. This data can then be supervised afterwards by a physician or a specialist to confirm or refute the findings. Also, if the arrythmia actually occurred, the healthcare professional can establish a course of action.

Apart from the characteristics mentioned above in the previous paragraphs, the method according to a first aspect of the invention may have one or several complementary characteristics among the following characteristics considered individually or in any technically possible combinations.

In one embodiment, the occurrence of the idiopathic arrythmia is determined by a first machine learning algorithm.

Thanks to this embodiment, the detection of the idiopathic arrythmia is more efficient by the use of big data analytics and artificial intelligence.

In one embodiment compatible with the previous embodiment, the first machine learning algorithm is a first neural network, the first neural network being trained on a first database comprising the at least one reference signal.

In one embodiment compatible with any of the preceding embodiments, the message is created using a decision tree based on the first signal and the information about the health status of the user.

Thanks to this embodiment, the decision tree helps the user to execute the best action given his current condition, for example: call an emergency service, schedule an appointment with a physician or a specialist, urge to go to the emergency department, etc. The decision tree also helps to direct the user to the best suited physician or specialist given the detected idiopathic arrythmia and the information he has provided.

In one embodiment compatible with the preceding embodiments, the message is an alert, wherein the alert is sent to an emergency service to provide medical assistance to the user when the user does not respond to the request of health status information after a predefined duration.

Thanks to this embodiment, the system can decide to call the emergency service for the user, considering that the user is not able to respond to the request of his/her health state information because he is unconscious.

In one embodiment compatible with any of the previous embodiments, the message is further based on an electronic medical record of the user.

Thanks to this embodiment, the system can decide the action to take based on previous medical examinations or clinical information relative to the user.

In one embodiment compatible with any of the previous embodiments, the electronic medical record of the user is updated with one or more of: the first signal, the determined occurrence of the idiopathic arrythmia, the information about the health state of the user and the emitted message.

Thanks to this embodiment, the electronical medical record of the user is kept updated with the detected idiopathic arrythmia for further analysis by the physician or the specialist.

In one embodiment compatible with any of the previous embodiments, the message comprises information for scheduling an appointment with a physician or specialist.

Thanks to this embodiment, the user is advised to schedule an appointment with the physician or the specialist when the idiopathic arrythmia is detected, for the physician or specialist to analyse the idiopathic arrythmia.

According to a second aspect of the invention, it is provided a method for predicting arrythmia when the arrythmia has already been detected in a user, comprising:
- Receiving a second signal acquired with the electrocardiogram sensor;
- Predicting an occurrence of the arrythmia based on a comparison between said second signal and at least one prediction reference signal; and
- When the occurrence of the arrythmia is predicted, sending a prediction message.

Thanks to this second aspect, it is possible to predict an anomalous event (arrhythmia) and the user can thus anticipate his actions based on this prediction, for example by avoiding any risks (i.e., pull over the car if they are driving), resting and calling the emergency service, the physician, or the specialist.

In one embodiment compatible with the previous embodiments, the occurrence of the arrythmia is predicted by a second machine learning algorithm.

Thanks to this embodiment, the prediction of the arrythmia is more efficient by the use of artificial intelligence.

In one embodiment compatible with the previous embodiment, the second machine learning algorithm is a second neural network, the second neural network being trained on a second database comprising the at least one prediction reference signal.

In one embodiment compatible with the previous three embodiments, the message is sent to the emergency service to anticipate an assistance to be provided to the user.

Thanks to this embodiment, a medical assistance to provide to the user can be anticipated before the occurring of the arrythmia.

In one embodiment compatible with the previous four embodiments, the electronic medical record of the user is updated with one more of: the second signal, the predicted occurrence of the arrythmia and the prediction message.

Thanks to this embodiment, the electronical medical record of the user is kept updated with the predicted arrythmia for further analysis by the physician or the specialist, which can then confirm or contradict the prediction.

According to a third aspect of the invention, it is provided a system for monitoring arrythmias of a user, comprising:
- An electrocardiogram sensor; and
- A first module configured to:
   - Receive a first signal acquired by the electrocardiogram sensor;
   - Determine an occurrence of an arrythmia in the first signal based on a comparison between said first signal and at least one reference signal; and
   - When the occurrence of the arrythmia is determined
      ∘ Request the user to provide information about his/her health state; and
      ∘ Send a message based on the first signal and the health state information.

In one embodiment, the system according to the third aspect further comprises a second module configured to:
- Receive a second signal acquired with the electrocardiogram sensor;
- Predict an occurrence of an arrythmia based on a comparison between said second signal and at least one prediction reference signal; and
- When the occurrence of the arrythmia is predicted, send a prediction message.

In one embodiment compatible with the previous embodiment, the electrocardiogram sensor is comprised in a wearable.

Thanks to these, the system is compatible with real time monitoring and long-term monitoring. The system is also light and cost-effective.

Advantageously, the system can be a smart device, for example a smartphone or a smartwatch, that is thus easy to wear or handle, not bulky and non-invasive.

According to a fourth aspect of the invention, it is provided a computer program product comprising instructions which, when the program is executed by a computer, causes the computer to carry out the method according to any of the first or second aspect of the invention.

According to a fifth aspect of the invention, it is provided a computer-readable medium comprising instructions which, when executed by a computer, cause the computer to carry out the method according to any of the first or second aspect of the invention

### BRIEF DESCRIPTION OF THE FIGURES

The figures are presented for information purposes only and in no way limit the invention.
- Figure 1 schematically represents an embodiment of the system according to the invention.
- Figure 2 is a flow chart of a method according to the invention.
- Figure 3 is a flow chart of another method according to the invention.

The invention and its various applications will be better understood by reading the following description and examining the accompanying figures.

### DETAILED DESCRIPTION OF THE PREFERRED EMBODIMENT

Some embodiments of systems and methods of the present invention are now described, by way of example only, and with reference to the accompanying drawings. The description is not to be regarded as restrictive but as illustrative in nature.

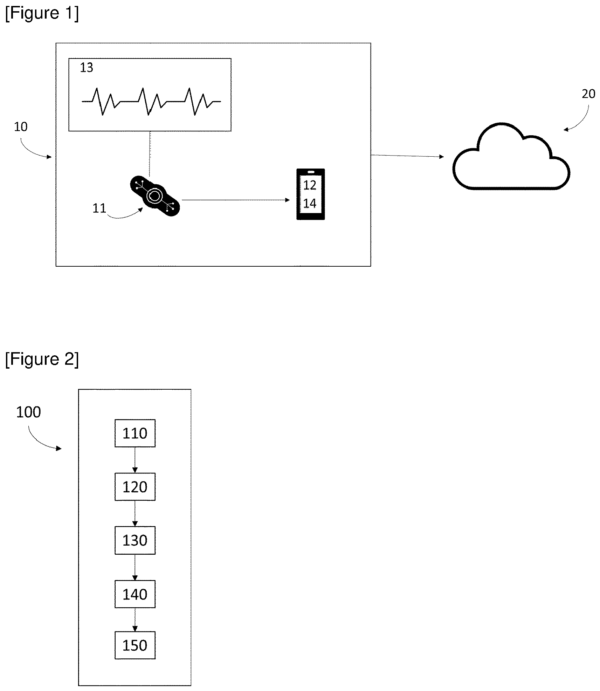

A first aspect of the invention relates to a system 10 for monitoring idiopathic arrythmias of a user of said system 10, as illustrated by the embodiment on figure 1.

The system 10 comprises an electrocardiogram sensor 11 and a first module 12.

The electrocardiogram sensor 11 is adapted to measure samples of an electrocardiogram signal 13 of the user. The electrocardiogram sensor 11 can be any type of electrocardiogram sensor and is worn by the user. For example, the electrocardiogram sensor 11 can be a patch fixed on a portion of the skin of the user. The electrocardiogram sensor 11 is preferably comprised in a smart device worn by the user. The electrocardiogram sensor 11 can also be comprised in a wearable or clothing; for example, it can be a T-shirt or a shirt comprising electrodes.

The first module 12 comprises means to perform computation tasks, for example a processing unit such as a microprocessor and a memory such as a volatile and/or non-volatile memory.

The first module 12 can be implemented in the smart device. Said smart device can also comprise a user interface which can be interacted with using a screen of said smart device. In the embodiment of figure 1, the smart device is a smartphone, but other smart devices, such as smartwatches can be used.

The electrocardiogram sensor 11 and the first module 12 are connected to each other, either by a wired or non-wired connection. For example, the first module 12, implemented in the smartphone, is connected to the electrocardiogram sensor 11 via a short-distance network, such as a Bluetooth network, or a long-distance network, such as a Wi-Fi network or mobile network. Wires can also be used to connect the smartphone to the electrocardiogram sensor 11.

The electrocardiogram sensor 11 and the first module 12, can be implemented in the same smart device, which is the case, for example, of a smartwatch.

The first module 12 is adapted to receive the readings of the electrocardiogram signal 13 acquired by the electrocardiogram sensor 11. In the following, for simplicity, by "electrocardiogram signal" is meant the readings of said electrocardiogram signal.

The first module 12 is also configured to continuously acquire the electrocardiogram signal 13 over time, for example by acquiring said electrocardiogram signal 13 during a sliding window of a predefined duration and stored on a portion of the memory used as a buffer.

In the following, for ease of understanding, the signal 13 acquired by the first module 12 is called the first signal 13.

The first module 12 is also configured to implement the steps of a method for detecting and analysing arrythmias, notably idiopathic arrythmias. Figure 2 schematically represents the chaining of the steps of the method 100. The method 100 comprises five consecutive steps, numbered from 110 to 150. The first module 12 therefore comprises instructions stored in its memory that, when executed by the processing unit, lead the processing unit to implement the method 100.

The first step 110 of the method 100 is a step of acquiring the first signal 13 by the electrocardiogram sensor 11. The first signal 13 can be acquired during the predefined duration of the sliding window at a first sampling frequency. The first signal 13 can be stored on the memory of the first module 12. The predefined duration is short enough to allow lower computation costs but is long enough to allow a robust analysis of said first signal 13. Preferably, the first signal 13 acquisition is continuously performed using the sliding window, which allows continuous real time processing of the first signal 13.

The second step 120 of the method 100 is a step of determining whether an idiopathic arrythmia is occurring in the first signal 13. This determination is performed by comparing the first signal 13 with one or more reference signals.

The reference signals are electrocardiogram signals previously acquired. The reference signals are acquired for a plurality of reference users, which can include the user. The reference signals can be of the same time length, shorter or longer than the first signal. The reference signals can be acquired at the first sampling frequency or a different one. The reference signals can be acquired with a same or a different electrocardiogram sensor. Each reference signal is tagged with a label indicating whether there is an arrythmia occurring in said reference signal. The reference signals can be the signals comprised in the MIT BIH Arrhythmia database or the PTB Diagnosis ECG database.

The comparison can be achieved by deriving a distance between the first signal 13 and each of the reference signals. The distance can be a difference or a sum of said first signal 13 with each of the reference signals. The distance can then be compared to a threshold that, when reached, indicates that there is an occurrence of the idiopathic arrythmia in the first signal 13.

Alternatively, the comparison is performed using a first machine learning algorithm. Preferably, the first machine learning algorithm is a first neural network trained on a first database comprising the reference signals. The first neural network takes as input the first signal 13 and provides as output if the idiopathic arrythmia occurs in the first signal 13. The output can be a discrete value or label, or it can be a continuous value. As an example, the first neural network is a convolutional neural network or a deep neural network. Implementation examples are provided in "Ali Haider Khan, Muzammil Hussain, Muhammad Kamran Malik, Arrhythmia Classification Techniques Using Deep Neural Network, Complexity, vol. 2021, ID 9919588, 2021".

The first neural network is preferably implemented in the first module 12 after its training using the first database. Alternatively, the first neural network is implemented on a cloud service 20. In this case, the first module 12 can transmit the first signal 13 to the cloud service 20 via a wired or wireless connection mean, for example via the mobile network used by the smart device. The output of the first neural network is then transmitted back to the first module 12 via the connection mean.

When the arrythmia occurrence is detected, the method 100 further proceeds to the third step 130 which is a step of requesting the user to provide information about his health state. This step requires the user to fill a form with questions about his current health condition. Questions are about the user health status: feeling well, dizzy, nauseous, cold, sweating, achy, etc. The user can also complement the form with relevant additional information.

The form to be filled by the user is designed by physicians and clinical specialists to gather relevant information regarding the occurrence of arrythmias. This helps the physician to understand the cause of the arrythmia and thus deliver a better care to the user.

After the gathering of the health status information from the user, the method 100 comprises a fourth step 140, which is a step of sending a message. The message is preferably created using a decision tree that considers the first signal 13 and the health status information of the user. In the embodiment of figure 1, the decision tree is implemented on the cloud service 20 and the message is sent from the cloud service 20.

The decision tree can produce several different decision outcomes. Among those, the decision tree can indicate, but is not limited to:
- Do nothing if the health status information and the first signal 13 indicate that the arrythmia detection is a false-positive; in this case, the message comprises the decision outcome that the detection was a false-positive;
- Advise the user to schedule an appointment with a physician or a specialist, or automatically schedule said appointment; the message then comprises the decision outcome to schedule the appointment; it can also comprise agenda availability of one or more physicians/specialists or the date of the automatically scheduled appointment;
- Advise the user to call an emergency department to request medical assistance; the message then comprises the decision outcome to call the emergency department; it can also comprise the local emergency phone number and/or the local emergency department address;
- Send an alert to an emergency service to provide a medical assistance to the user; the message can then comprise data about the user, such as their current location, their identity data, a report comprising the health status information of the user and/or the first signal 13.

In the case the user has not answered the form of the previous step, the decision tree can lead to a decision outcome to send the alert to the emergency department, after a predefined time, assuming that the user is unconscious. The predefined time can be set by one or more physicians/specialists based on their medical knowledge. All the decision outcomes of the decision trees are implemented and carried out by the first module 12.

Advantageously, the completeness of the decision tree provides support to distinguish between the idiopathic arrythmia and another symptom and, therefore, to direct the user to the best clinical service according to said symptom.

In some embodiments, the message is further generated based on an electronic medical record of the user comprising clinical data of said user from previous medical examination and/or previous medical events, including previous idiopathic arrythmia detection by the system 10. The electronic medical record can be hosted on a medical cloud service dedicated to store the electronic medical records of a plurality of users. In this case, the system 10 and/or the cloud service 20 can be authorized to access the medical cloud service to access the electronic medical record of the user. The electronic medical record can be accessed for example by using Health Level Seven (HL7) communication standards.

The method 100 comprises a fifth step 150, which is an optional step of updating the electronic medical record of the user. The update can be to add one more of: the first signal, the determined occurrence of the idiopathic arrythmia, the information about the health status of the user and the message sent to the electronic medical record. In this case, the system 10 and/or the cloud service can be authorized to access the medical cloud service to write on the electronic medical record.

In an embodiment, the system 10 can comprise a second module 14. The second module 14 comprises means to perform computation tasks, for example a processing unit such as a microprocessor and a memory such as a volatile and/or non-volatile memory. The second module 14 can also be implemented in a smart device, alike the first module 12. The second module 14 can be implemented in the same smart device as the first module 12.

The electrocardiogram sensor 11 and the second module 14 are connected to each other, either by a wired or non-wired connection. For example, the second module 14, implemented in the smartphone, is connected to the electrocardiogram sensor 11 via a short-distance network, such as a Bluetooth network, or a long-distance network, such as a Wi-Fi network or mobile network. Wires can also be attached to connect the smartphone to the electrocardiogram sensor 11.

The electrocardiogram sensor 11 and the second module 14, can be implemented in the same smart device, for example, the same smartwatch comprising the first module 12.

The second module 14 is also configured to receive the electrocardiogram signal 13 acquired by the electrocardiogram sensor 11.

The second module 14 is also configured to continuously acquire the electrocardiogram signal 13 over time, for example by acquiring said electrocardiogram signal 13 during a sliding window of a predefined duration and stored on a portion of the memory used as a buffer.

In the following, for ease of understanding, the signal 13 acquired by the second module 14 is called the second signal 13.

The first module 12 and the second module 14 can be implemented concurrently by the system 10, preferably by the smart device, or only one of them can be implemented by said system 10, preferably by the smart device. Alternatively, the second module 14 is the first module 12.

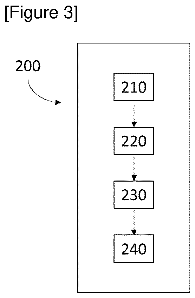

The second module 14 is also configured to implement the steps of a method for predicting arrythmias. Figure 3 schematically represents the chaining of the steps of the prediction method 200. The prediction method 200 comprises four consecutive steps, numbered from 210 to 240. The second module 14 therefore comprises instructions stored in its memory that, when executed by the processing unit, lead the processing unit to implement the prediction method 200.

The second module 14 and the prediction method 200 are preferably used to predict arrythmia occurrences for users for whom the cause of the idiopathic arrythmia has already been discovered. The discovery of the cause of the idiopathic arrythmia can be achieved thanks to any approach for assisting the physician/specialist in diagnosing the cause of idiopathic arrythmias, such as the method 100 described above.

The first step 210 of the prediction method 200 is a step of acquiring the second signal 13 by the electrocardiogram sensor 11. The second signal 13 can be acquired during a second predefined duration of the sliding window at a second sampling frequency. The second signal 13 can be stored on the memory of the second module 14. The second predefined duration is short enough to allow lower computation costs but is long enough to allow a robust analysis of said second signal 13. Preferably, the second signal 13 acquisition is continuously performed thanks to the sliding window, which allows continuous real time processing of the second signal 13. The second predefined duration can be the same as the predefined duration.

The second step 220 of the prediction method 200 is a step of predicting whether an arrythmia is going to occur. This prediction is performed by comparing the second signal 14 with one or more prediction reference signals.

The prediction reference signals are electrocardiogram signals previously acquired. The prediction reference signals are acquired for a plurality of prediction reference users, which can include the user. The prediction reference signals can be of the same time length, shorter or longer than the second signal 13. The reference signals can be acquired at the second sampling frequency or a different one. The prediction reference signals can be acquired with a same or a different electrocardiogram sensor 11. Each prediction reference signal is labelled with a label indicating whether an arrythmia is going to occur. The prediction can be made several seconds to several hours in advance of the actual occurring of the arrythmia, depending on the prediction method used. The reference signals can be the signals comprised in the MIT BIH Arrhythmia database or the PTB Diagnosis ECG database.

The comparison can be achieved by deriving a distance between the second signal 13 and each of the prediction reference signals, the same way as it is done at step 120 of the method 100. The prediction distance can then be compared to a threshold which, when reached, indicates that the arrythmia is going to happen.

The prediction distance can also indicate when the arrythmia is going to happen. For example, it is possible to produce a time to occurrence, i.e., when the arrythmia occurs, thanks to the label attached to each of the prediction reference signals. Indeed, this label can also comprise a delay before occurring of the arrythmia. The time to occurrence can then be the delay before occurrence of the arrythmia from the label attached to the prediction reference signal for which the computed distance is the shortest. The time to occurrence and the delay before occurrence of the arrythmia are times given in time unit, for example in seconds, minutes, hours, days, weeks, etc.

The prediction distance can also indicate a severity of the soon to occur arrythmia based on a reference severity of the arrythmia in the label attached to each of the prediction reference signals.

Alternatively, the comparison is performed using a second machine learning algorithm. Preferably, the second machine learning algorithm is a second neural network trained using a second database comprising the prediction reference signals. The second neural network takes as input the second signal 13 and provides as output if an arrythmia is going to occur. The output can be a discrete value or label, or it can be a continuous value, to indicate a probability of occurrence. The second neural network can also provide as output the time to occurrence and/or the severity of the soon to occur arrythmia. As an example, the second neural network is a convolutional neural network or a deep neural network. Implementation examples are provided in "Abdoul-Dalibou Abdou, Ndeye Fatou Ngom, Oumar Niang, Electrocardiograms patterns analysis using Artificial Neural Network and non-linear regression, in proceedings of CARI 2018, Stellenbosch, South Africa, October 2018".

The second neural network is preferably implemented in the second module 14 after its training using the second database. Alternatively, the second neural network is implemented on the cloud service 20. In this case, the second module 14 is configured to transmit the second signal 13 to the cloud service 20 via a wired or wireless connection mean, for example via the mobile network used by the smart device. The outputs of the second neural network are then transmitted back to the second module 14 via the connection mean.

Advantageously, the second neural network can learn on the fly from the prediction it has made by making a physician or a specialist confirm or contradict the prediction. This confirmation and/or contradiction can be carried out afterwards, for example when the user meets the physician/specialist at the emergency service or during a scheduled appointment.

The prediction method 200 then comprises a third step 230 of sending a prediction message. The message is preferably sent to the emergency service to anticipate an assistance to be provided to the user. The message can comprise data about the user, such as their current location, their identity data and/or the second signal 13. If the time to occurrence is greater than a predefined delay, for example in more than two hours, the message is sent to the emergency service to indicate that the user is coming to the location of the emergency service, and the message is also sent to the user to advise him/her to go to the emergency service. In this case, the message to the user can comprise the local emergency phone number and/or the local emergency service address, and the message to the emergency service can comprise the identity data of the user and/or the second signal 13.

In some embodiments, the method 200 further comprises a fourth step 240 of updating the electronic medical record of the user by adding to said record one more of: the second signal 13, the predicted occurrence of the arrythmia, the time to occurrence, the occurrence probability, and the prediction message. In this case, the system 10 and/or the cloud service 20 can also be authorized to access the medical cloud service to write the data to the electronic medical record.

Preferentially, when the system 10 is a smart device, such as a smartphone or a smartwatch, the method 100 and/or the method 200 are implemented through an application installed on the smart device and integrates an application programming interface adapted to collect the electrocardiogram signal 13 and the information on health status of the user in a data lake.

In some embodiments, the user can execute any of the method 100 or the prediction method 200 at their will, for example because he/she is feeling unwell and thinks that the arrythmia is occurring or is going to happen.

In some embodiments, the detection method 100 and the prediction method 200 are implemented on the cloud 20 service instead of on the first module 12. In this case, the first module 12 is configured to receive the first signal 13 and to send the first signal 13 to the cloud service 20.

## Claims

1. Method (100) for detecting idiopathic arrythmias of a user, comprising:
- Receiving (110) a first signal (13) acquired by an electrocardiogram sensor (11);
- Determining (120) an occurrence of idiopathic arrythmia in the first signal (13) based on a comparison between said first signal (13) and at least one reference signal; and
- When the occurrence of the idiopathic arrythmia is determined:
• Requesting (130) the user to provide information about his/her health status; and
• Sending (140) a message based on the first signal (13) and the information about the health state of the user.

2. Method (100) according to any of the preceding claims, wherein the occurrence of the idiopathic arrythmia is determined by a first machine learning algorithm.

3. Method (100) according to the preceding claim, wherein the first machine learning algorithm is a first neural network, the first neural network being trained on a first database comprising the at least one reference signal.

4. Method (100) according to any of the preceding claims, wherein the message is created using a decision tree based on the first signal (13) and the information about the health status of the user.

5. Method (100) according to any of the preceding claims, wherein the message is an alert, and wherein the alert is sent to an emergency service to provide medical assistance to the user when the user does not respond to the request of the health status information after a predefined duration.

6. Method (100) according to any of the preceding claims, wherein the message is further based on an electronic medical record of the user.

7. Method (100) according to the preceding claim, wherein the electronic medical record of the user is updated (150) with one or more of: the first signal (13), the determined occurrence of the idiopathic arrythmia, the information about the health state of the user and the emitted message.

8. Method (200) for predicting arrythmias when this arrythmia has already been detected in a user, comprising:
- Receiving (210) a second signal (13) acquired with the electrocardiogram sensor (11);
- Predicting (220) an occurrence of the arrythmia based on a comparison between said second signal (13) and at least one prediction reference signal; and
- When the occurrence of the arrythmia is predicted, sending (230) a prediction message.

9. Method (100) according to claim 8, wherein the occurrence of the arrythmia is predicted by a second machine learning algorithm.

10. Method (100) according to claim 9, wherein the second machine learning algorithm is a second neural network, the second neural network being trained on a second database comprising the at least one prediction reference signal.

11. Method (100) according to any of claims 8 to 10, wherein the message is sent to the emergency service to anticipate an assistance to be provided to the user.

12. Method (100) according any of claims 8 to 11, wherein the electronic medical record of the user is updated (240) with one more of: the second signal (13), the predicted occurrence of the arrythmia and the prediction message.

13. A system (10) for monitoring arrythmias of a user, comprising:
- An electrocardiogram sensor (11); and
- A first module (12) configured to:
• Receive (110) a first signal (13) acquired by the electrocardiogram sensor (11);
• Determine (120) an occurrence of an arrythmia in the first signal (13) based on a comparison between said first signal (13) and at least one reference signal; and
• When the occurrence of the arrythmia is determined:
∘ Request (130) the user to provide information about his/her health state; and
∘ Send (140) a message based on the first signal (13) and the health state information.

14. System (10) according to the preceding claim, further comprising a second module (14) **characterized in that** it is configured to:
- Receive (210) a second signal (13) acquired with the electrocardiogram sensor (11);
- Predict (220) an occurrence of an arrythmia based on a comparison between said second signal (13) and at least one prediction reference signal; and
- When the occurrence of the arrythmia is predicted, send (230) a prediction message.

15. System (10) according to any of claims 13 and 14, wherein the electrocardiogram sensor (11) is comprised in a wearable.
